(19) Europäisches Patentamt
European Patent Office
Office européen des brevets

(11) **EP 3 061 395 A1**

(12) **EUROPEAN PATENT APPLICATION**
published in accordance with Art. 153(4) EPC

(43) Date of publication:
**31.08.2016 Bulletin 2016/35**

(51) Int Cl.:
***A61B 5/107*** *(2006.01)*   ***G06T 1/00*** *(2006.01)*

(21) Application number: **14856427.1**

(86) International application number:
**PCT/JP2014/075876**

(22) Date of filing: **29.09.2014**

(87) International publication number:
**WO 2015/060070 (30.04.2015 Gazette 2015/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **21.10.2013 JP 2013218172**

(71) Applicant: **Konica Minolta, Inc.
Tokyo 100-7015 (JP)**

(72) Inventor: **MATSUDA, Shinya
Tokyo 100-7015 (JP)**

(74) Representative: **Gille Hrabal
Brucknerstrasse 20
40593 Düsseldorf (DE)**

(54) **ORGAN IMAGE CAPTURE DEVICE**

(57)    An organ image capture device (1) comprises an imaging unit (3), a contour extraction unit (6), and a detection unit (7). The imaging unit (3) images an organ of a living body and obtains an image. The contour extraction unit (6) extracts a contour of the organ from the image obtained by the imaging unit (3). The detection unit (7) calculates a fitted curve approximating the contour of the organ or a part of the contour extracted by the contour extraction unit (6) and detects unevenness of the contour on the basis of the degree of correlation between the contour and the fitted curve.

FIG.2

## Description

## Technical Field

The present invention relates to an organ imaging device which detects dents and bumps in a contour line of an organ of a living body from an image obtained by imaging the organ.

## Background Art

In Oriental medicine, a method of diagnosis (tongue diagnosis) is known in which the condition of the human tongue is inspected to diagnose health condition and disease condition. In tongue diagnosis, an examinee's physical condition and healthiness are examined based on the color and shape of the tongue.

One item of diagnosis in tongue diagnosis is whether or not there is a tooth mark in a peripheral part of the tongue. Poor water metabolism in cells causes the tongue to swell. This brings a peripheral part of the tongue into contact with teeth, producing tooth marks. In Oriental medicine, such poor metabolism is called "water stagnation", which is believed to result from dysfunction of the circulatory system or the digestive system. As the dysfunction deteriorates, dizziness, lassitude, and headaches may result.

Such diagnosis is practiced by specialized physicians, who, however, rely on experience and intuition. Thus, diagnoses tend to vary from one practitioner to another and be far from objective. Moreover, obscure memories of past condition hamper an objective grasp of changes in condition.

As a solution, there have been proposed systems in which a subject is imaged with a digital camera and, from the taken image, features are quantified and recorded to make a diagnosis. For example, according to Patent Document 1, from a taken image of the tongue, the center of gravity of the tongue is found; then, for each of a plurality of points on the contour line of the tongue, the distance from the center of gravity to the point is calculated, and the sum of the differences between every two adjacent distances is calculated. When the sum of the differences is large, it is determined that there is a tooth mark and it is severe; when the sum of the differences is small, it is determined that there is hardly a tooth mark and it is mild.

On the other hand, according to Patent Document 2, a binarized image of the tongue is scanned along a plurality of scan lines, and it is checked whether or not the width of the peak that represents the contour of the tongue varies irregularly from one scan line to another to check for a tooth mark. Specifically, if there is no tooth mark on the tongue, the width of the peak (the width of the shadings defining the contour line of the tongue) is constant among scan lines; if there is a tooth mark, because it appears black due to shadings on the surface, the width of the peak varies irregularly among scan lines. Thus, by detecting variation in the width of the peak, it is possible to detect whether or not there is a tooth mark.

## List of Citations

## Patent Literature

Patent Document 1: Japanese Patent Application Publication No. 2005-137756 (See paragraphs [0075] to [0078], Figs. 6, 7, etc.)
Patent Document 2: Japanese Patent Application Publication No. 2009-28058 (See paragraphs [0050] to [0051], Fig. 7, etc.)

## Summary of the Invention

## Technical Problem

The exterior shape of the tongue varies among individuals, like thin or thick, round or square, and so forth. Accordingly, the distances from the center of gravity of the tongue to points on the contour line vary not only according to whether or not there is a tooth mark but with the exterior shape of the tongue. Thus, with the method disclosed in Patent Document 1, measured values (the differences between every two adjacent distances) vary with the exterior shape of the tongue. Thus, this method cannot be said to allow precise detection of a tooth mark.

On the other hand, shadings on the tongue surface are produced only by a severe tooth mark on the tongue surface (large dents and bumps in the contour line); that is, a mild tooth mark (small dents and bumps in the contour line) does not produce shadings on the tongue surface. Accordingly, with the method disclosed in Patent Document 2, which detects a tooth mark based on shadings, when the tongue only has a mild tooth mark, it is not possible to detect it, resulting in low tooth-mark detection precision.

To improve the precision of diagnosis based on a tooth mark, that is, diagnosis based on dents and bumps in the contour line of an organ, a detection method is sought that allows precise detection of dents and bumps in a contour line irrespective of the exterior shape of an organ and even when the contour line has only small dents and bumps.

Devised to overcome the inconveniencies mentioned above, the present invention aims to provide an organ imaging device that can precisely detect dents and bumps in a contour line irrespective of the exterior shape of an organ and even in a case where the contour line only has small dents and bumps.

## Means for Solving the Problem

According to one aspect of the present invention, an organ imaging device including an imager for imaging an organ of a living body to acquire an image of the organ further includes: a contour line extractor for extracting a contour line of the organ from the image acquired by the imager; and a detector for finding an approximate curve that approximates the contour line, or part of the contour line, of the organ extracted by the contour line extractor, and detecting dents and bumps in the contour line based on the degree of correlation between the contour line and the approximate curve.

## Advantageous Effects of the Invention

With the above configuration, by detecting dents and bumps in a contour line based on the degree of correlation between the contour line of an organ and an approximate curve, it is possible to precisely detect dents and bumps in the contour line irrespective of the exterior shape of the organ and even in a case where the contour line only has small dents and bumps.

## Brief Description of Drawings

[Fig. 1] is a perspective view showing an exterior view of an organ imaging device according to one embodiment of the present invention;

[Fig. 2] is a block diagram showing an outline of a configuration of the organ imaging device;

[Fig. 3] is a diagram illustrating an example of an edge extraction filter used in a contour line extractor in the organ imaging device;

[Fig. 4] is a diagram illustrating the illumination angle of an imaging object with respect to the organ imaging device;

[Fig. 5] is a diagram illustrating an example where the contour line of the tongue extracted by the contour line extractor is approximated by a plurality of polynomial equations;

[Fig. 6] is a diagram illustrating contour lines and approximate curves for a plurality of tongues with different exterior shapes;

[Fig. 7] is a diagram illustrating a taken image of the tongue with tooth marks;

[Fig. 8] is a diagram illustrating a tongue contour line extracted from the taken image in Fig. 7;

[Fig. 9] is a diagram showing the tongue contour line in Fig. 8, an approximate curve approximating it, a polynomial equation expressing the approximate curve, and a determination coefficient $R^2$;

[Fig. 10] is a plot of y coordinate differences between the tongue contour line in Fig. 8 and the approximate curve;

[Fig. 11] is a diagram illustrating the relationship between actual diagnoses, as made by a *Kampo* doctor on the tongues of a plurality of examinees, and the degree of correlation;

[Fig. 12] is a plot showing the relationship between diagnoses by a *Kampo* doctor and the determination coefficient or the maximum value of coordinate differences;

[Fig. 13] is a plot showing the relationship between diagnoses by a *Kampo* doctor and the sum of coordinate differences;

[Fig. 14] is a flow chart showing the flow of operation in the organ imaging device; [Fig. 15] is a diagram illustrating other examples of taken images of the tongue according to another embodiment of the present invention;

[Fig. 16] is a diagram illustrating a contour line of the tongue (including a tip part of it) and an approximate curve approximating it; and

[Fig. 17] is a diagram illustrating part of a contour line of the tongue (excluding a tip part of it) and an approximate curve approximating it.

**Description of Embodiments**

<Embodiment 1>

Hereinafter, an embodiment of the present invention will be described with reference to the accompanying drawings. In the present description, a range of values from A to B is assumed to include the lower limit A and the upper limit B.

[Overcall Configuration of an Organ Imaging Device]

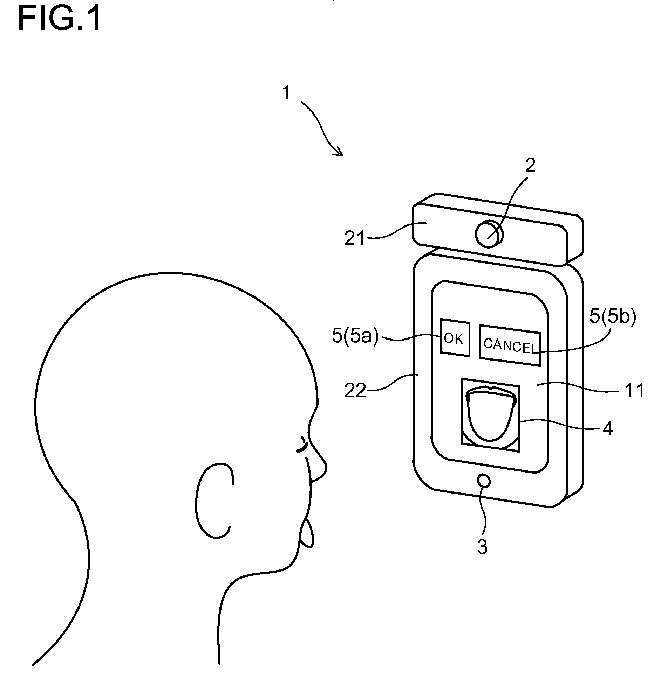

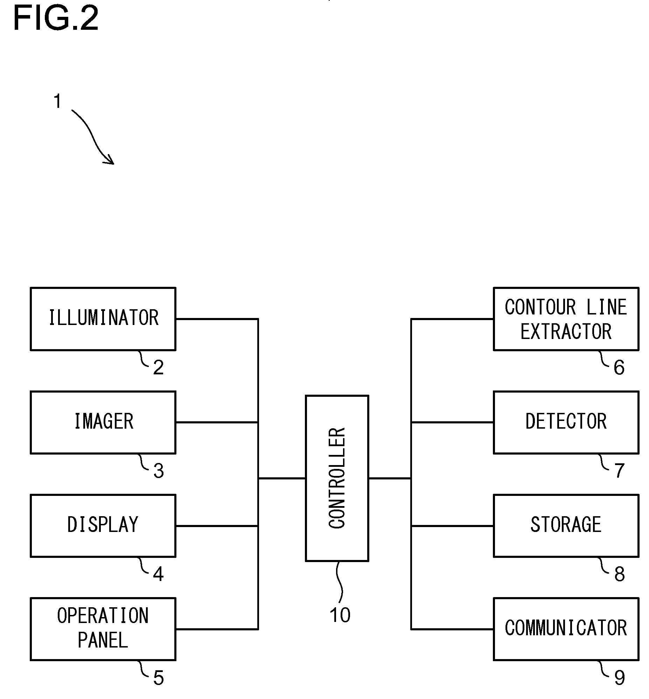

Fig. 1 is a perspective view showing an exterior appearance of an organ imaging device 1 according to one embodiment of the present invention, and Fig. 2 is a block diagram showing an outline of a configuration of the organ imaging device 1. The organ imaging device 1 is used to image an organ of a living body to extract information needed for diagnosis. The imaging object can be an organ of a living body (e.g., a tongue or an eye) and an area surrounding it (e.g., an area around a tongue, or an area under an eye). The following description deals with an example where the imaging object is the tongue as an organ of a living body.

The organ imaging device 1 includes an illuminator 2, an imager 3, a display 4, an operation panel 5, a contour line extractor 6, a detector 7, a storage 8, a communicator 9, and a controller 10. The illuminator 2 is provided in a housing 21, and the blocks other than the illuminator 2 (e.g., the imager 3, the display 4, and the operation panel 5) are provided in a housing 22. The housings 21 and 22 are coupled together so as to be rotatable relative to each other, but do not necessarily have to be so: one of them may be completely secured to the other. The illuminator 2 and the other blocks may be provided in a single housing. The organ imaging device 1 may be configured as a multifunction portable information terminal.

The illuminator 2 is configured as a light that illuminates an imaging object from above. The illuminator 2 includes a light source that emits light of a daylight color, such as a xenon lamp, for improved color rendering. The brightness of the light source varies depending on the sensitivity of the imager 3 and the distance to the imaging object; the brightness can be, for example, such as to provide an illumination of 1000 to 10000 lx at the imaging object. The illuminator 2 includes, in addition to the light source, a lighting circuit and a dimming circuit, and is controlled according to instructions from the controller 10 so as to be lit, extinguished, and dimmed.

The imager 3 images an organ of a living body to acquire an image of it under the illumination of the illuminator 2, and includes an imaging lens and an area sensor (image sensor). The aperture of the imaging lens (the fastness of the lens), the shutter speed, and the focal length are so set that the imaging object is in focus over its entire area. For example, the f-number can be set at 16, the shutter speed can be set at 1/120 seconds, and the focal length can be set at 20 mm.

The area sensor comprises, for example, an image sensor such as a CCD (charge-coupled device) image sensor or a CMOS (complementary metal oxide semiconductor) image sensor, and its sensitivity, resolution, etc. are so set that the color and shape of the imaging object can be detected satisfactorily. For example, the sensitivity can be set at 60 db, and the resolution can be set at 10 megapixels.

The imaging by the imager 3 is controlled by the controller 10. The imager 3 further includes, in addition to the imaging lens and the area sensor, a focusing mechanism, an aperture mechanism, a drive circuit, an A/D conversion circuit, etc., of which none is illustrated, and is controlled according to instructions from the controller 10 in terms of focus, aperture, A/D conversion, etc. The imager 3 acquires, as the data of a taken image, data comprising, for example, eight bits, representing a value from 0 to 255, for each of red (R), green (G), and blue (B) per pixel.

The display 4 includes a liquid crystal panel, a backlight, a lighting circuit, and a control circuit, of which none is illustrated, and displays the image acquired by the imaging by the imager 3 according to instructions from the controller 10. The display 4 can also display information (e.g., the result of diagnosis performed by an external medical facility based on information transmitted to it) acquired from outside via the communicator 9.

The operation panel 5 comprises an input device from which to instruct the imager 3 to perform imaging, and includes an OK button (TAKE IMAGE button) 5a and a CANCEL button 5b. In Embodiment 1, the display 4 and the operation panel 5 are constituted by a single touch panel display device 11, and separate display areas are provided on the touch panel display device 11, one for the display 4 and the other for the operation panel 5. The operation panel 5 may be configured as any other input device than the touch panel display device 11 (the operation panel 5 may be provided anywhere else than inside the display area of the touch panel display device 11).

The contour line extractor 6 extracts a contour line of an organ from an image acquired by the imager 3. In Embodiment 1, the contour line extractor 6 extracts the contour line of the tongue as an organ based on a luminance edge in the taken image (a part of the image where luminance changes sharply).

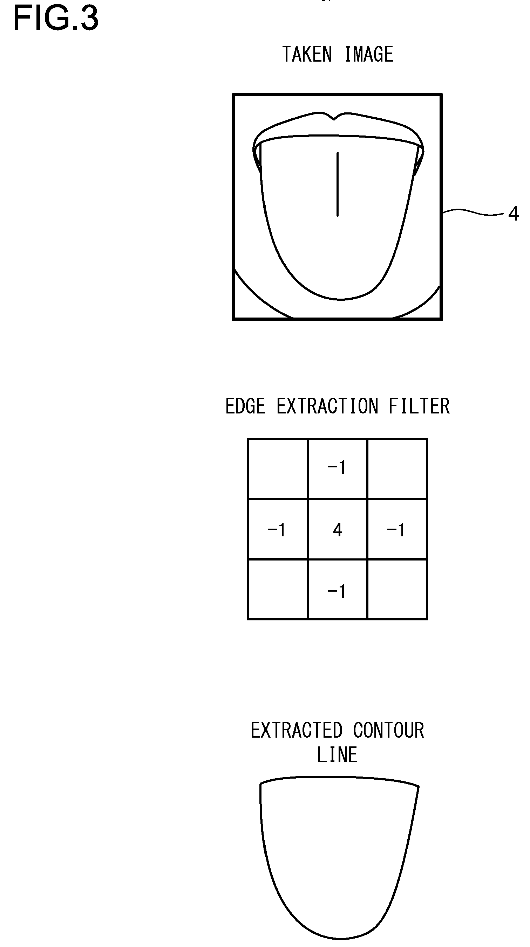

A luminance edge can be extracted, for example, by use of an edge extraction filter as shown in Fig. 3. An edge extraction filter is a filter that gives weights to pixels near a pixel of interest when performing first-order differentiation (when calculating differences in image data between neighboring pixels). By use of such an edge extraction filter, for example, with respect to the green image data of each pixel in the taken image, differences in image data are calculated between the pixel of interest and neighboring pixels, and those pixels which yield differences exceeding a predetermined threshold value are extracted; in this way, pixels that constitute a luminance edge can be extracted. Around the tongue, its shadow produces brightness differences; thus, by extracting pixels that constitute a luminance edge in the manner described above, it is possible to extract the contour line of the tongue. Here, green image data is used in the calculation because it has the greatest influence on luminance; red or blue image data may be used instead.

The detector 7 includes an unillustrated processor, and detects information necessary for diagnosis from an image acquired by the imager 3. In particular, in Embodiment 1, the detector 7 detects dents and bumps in the contour line of the tongue from the taken image, thereby to detect a tooth mark on the tongue. This will be described in detail later.

The storage 8 comprises a memory that stores the data of images acquired by the imager 3, information acquired by the contour line extractor 6 and the detector 7, information received from outside, etc. The communicator 9 comprises an interface for transmitting image data and information as mentioned above to outside via a communication network (which may be wired or wireless), and for receiving information from outside. The controller 10 controls the operation of relevant blocks in the organ imaging device 1, and comprises, for example, a CPU (central processing unit) and a memory, with programs for controlling different blocks stored in the latter.

[Examples of Arrangement of the Illuminator and the Imager]

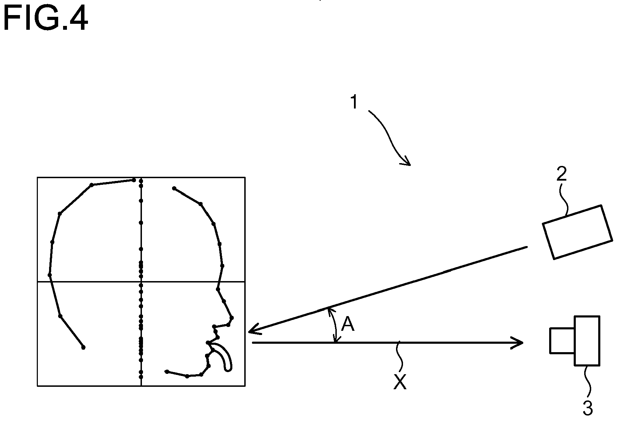

Fig. 4 is a diagram illustrating the illumination angle of an imaging object with respect to the organ imaging device 1. As shown there, the imager 3 is arranged right in front of the imaging object (the tongue or the face). The illuminator 2 is so arranged as to illuminate the imaging object, for example, at an angle A of 0° to 45° relative to the imaging optical axis X of the imager 3, which passes through the imaging object. The imaging optical axis X denotes the optical axis of the imaging lens provided in the imager 3.

Here, when illumination is applied at a large angle A, the shadow of the upper lip reduces the area over which the tongue can be imaged. Conversely, when illumination is applied at a small angle A, specular reflection causes severe color clipping. Out of these considerations, a preferred range of the angle A for illumination is from 15° to 30°.

[Detecting Dents and Bumps in a Contour Line]

Next, how the detector 7 detects dents and bumps in the contour line of the tongue will be described in detail. The detector 7 finds an approximate curve that approximates the contour line of the tongue extracted from a taken image by the contour line extractor 6, and detects dents and bumps in the contour line based on the degree of correlation between the contour line and the approximate curve. The approximate curve can be found by a well-known method (e.g., the least-square method).

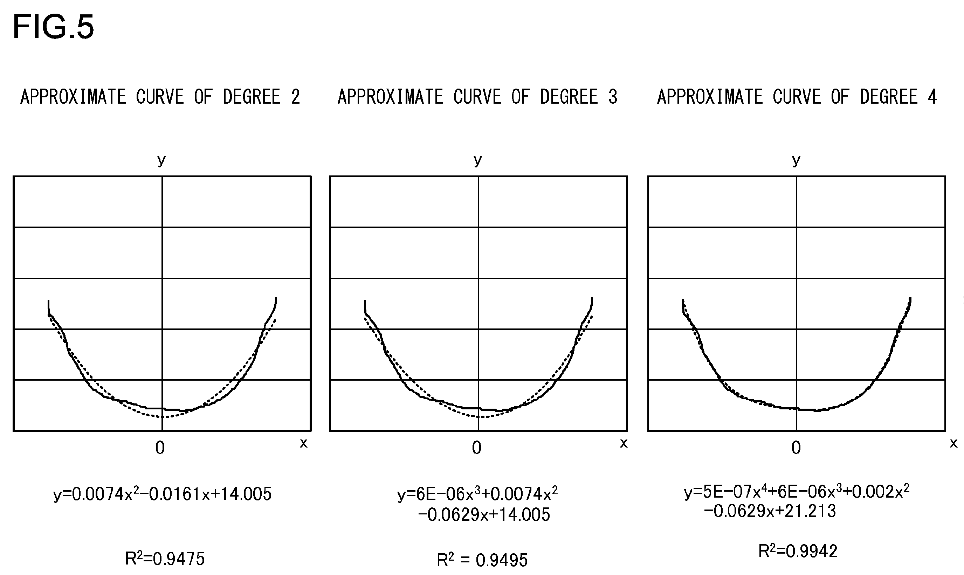

Fig. 5 shows an example in which the lower half of the tongue contour line extracted by the contour line extractor 6 is approximated by polynomial equations by use of the least-square method. In the diagrams, a solid line represents the extracted contour line of the tongue, and a broken line represents an approximate curve expressed by a polynomial equation. In a coefficient in an approximate curve, the notation "E-n" stands for "$\times 10^{-n}$" (the same applies equally to other drawings). The diagrams reveal the following: using a polynomial equation of degree 2 or more as an approximate curve yields 0.94 or more as a determination coefficient $R^2$ (which will be described in detail later) which indicates the degree of correlation between the tongue contour line and the approximate curve, and as the degree of the polynomial equation is increased from 2 to 3 to 4, the determination coefficient $R^2$ increases, and the approximate curve becomes increasingly close to the contour line.

Here, each approximate curve in Fig. 5 is expressed by the polynomial equation that gives the highest degree of correlation with the contour line among polynomial equations of the same maximum degree. For example, the approximate curve of degree 2 there is expressed by a polynomial equation that gives the highest degree of correlation (determination coefficient $R^2$) with the contour line among a plurality of polynomial equations expressed by equations of degree 2. Accordingly, in the example in Fig. 5, as an approximate curve that approximates the contour line, there exists no approximate curve of degree 2 that gives a determination coefficient $R^2$ greater than 0.9475. Likewise, there exists no approximate curve of degree 3 that gives a determination coefficient $R^2$ greater than 0.9495, and there exists no approximate curve of degree 4 that gives a determination coefficient $R^2$ greater than 0.9942.

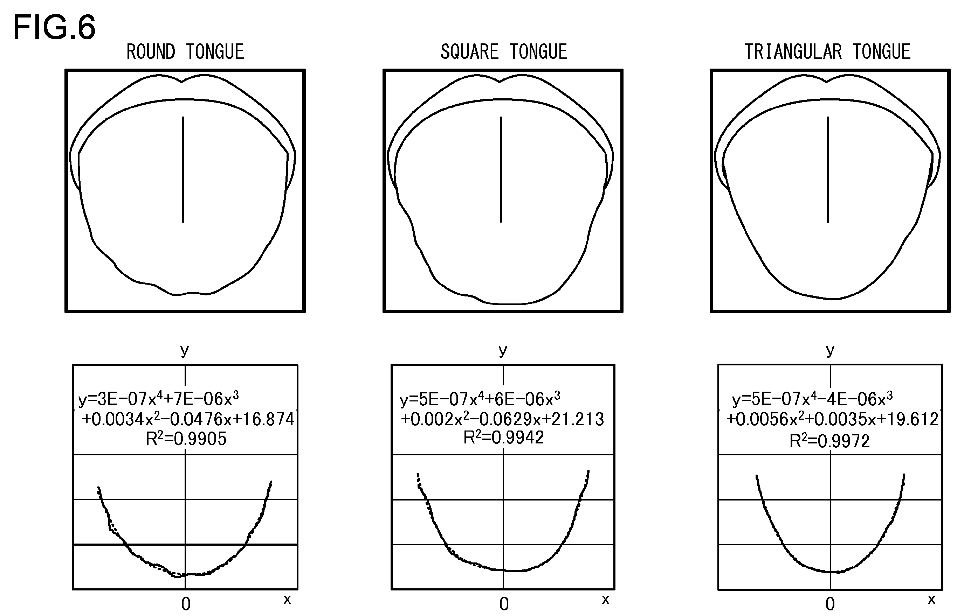

Through an analysis of a large number of sample images, the present inventor has found out that, by use of a polynomial equation of degree 4 or more as an approximate curve, it is possible to satisfactorily approximate the exterior shape of individual tongues despite differences in the tongue's exterior shape among individuals. Fig. 6 shows an example where, for each of tongues with different shapes, its contour line is approximated by an approximate curve expressed by an equation of degree 4 (a solid line representing the contour line, a broken line representing the approximate curve). In the diagrams, the determination coefficient $R^2$ indicating the degree of correlation between the tongue contour line and the curve approximating it (an equation of degree 4) is greater than 0.99 for all of a round, a square, and a triangular tongue. Thus, it can be concluded that, by approximating the tongue contour line with an approximate curve expressed by an equation of degree 4, it is possible to approximate the exterior shape of the tongue for most tongues despite differences in the tongue's exterior shape among individuals.

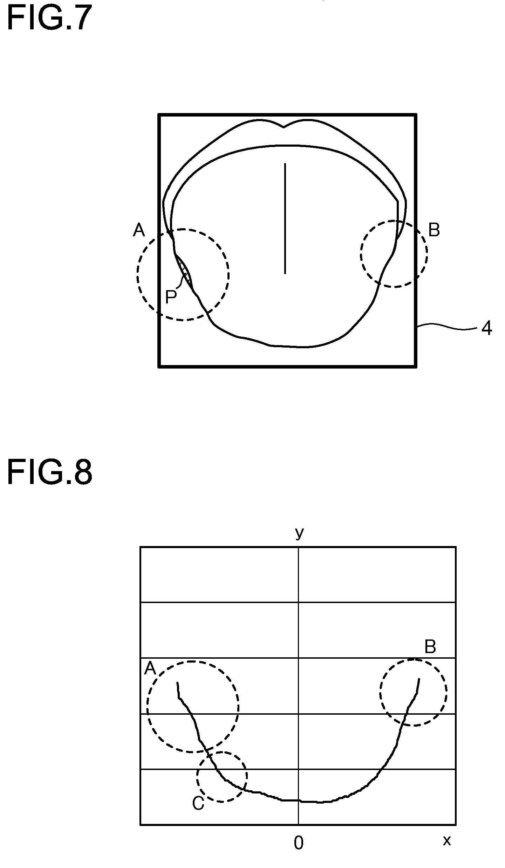

On the other hand, Fig. 7 shows a taken image of the tongue with a tooth mark. In a left-side part, part A, of the tongue and in a right-side part, part B, of the tongue, tooth marks are seen respectively. Of the two tooth marks, the one in part A is severe (with larger dents and bumps), and produces shadings P on the tongue surface. On the other hand, the tooth mark in part B is mild (with small dents and bumps), and produces no shadings on the tongue surface. Thus, with the conventional method that relies on shadings to check for a tooth mark (Patent Document 2), it is not possible to detect a mild tooth mark like the one in part B, leading to low tooth-mark detection precision.

Fig. 8 shows the tongue contour line extracted from the taken image of the tongue in Fig. 7. In parts corresponding to parts A and B in Fig. 7 (here again identified as parts A and B as in Fig. 7 for convenience's sake), rough dents and bumps are seen; in addition, another bump is seen in part C. In this example, even with a healthy examinee, with no tooth marks in parts A and B, the bump in part C is seen; thus the bump is considered to be ascribable not to a tooth mark but to the intrinsic shape of the tongue. With the conventional method in which dents and bumps in the tongue contour line itself are measured based on differences among the distances from points on the contour line to the center of gravity of the tongue (Patent Document 1), the differences change also in part C. Thus, even a healthy tongue with no tooth mark may be erroneously diagnosed to have a tooth mark. This is believed to lead to low tooth-mark detection precision.

As a solution, in Embodiment 1, the contour line of the tongue is approximated by an approximate curve, and based on the degree of correlation between the contour line and the approximate curve, dents and bumps in (the smoothness of) the contour line are detected, and thereby a tooth mark on the tongue is detected. The approximate curve is a smooth curve with no fine dents or bumps, and thus it can be concluded that, the closer the contour line is to the approximate curve, the smoother the contour line is, and the fewer tooth marks are. That is, the higher the degree of correlation between the contour line and the approximate curve is, the fewer tooth marks are; the lower the degree of correlation, the more tooth marks are.

As indices that indicate the degree of correlation between the two, there have been proposed several indices, of which the following three the present inventor has confirmed to comfortably agree to diagnoses made by a *Kampo* doctor (*Kampo* is a Japanese version of ancient Chinese medicine).

A first index that indicates the degree of correlation is a determination coefficient $R^2$ given by the formula below.

$$R^2 = 1 - \{(\Sigma(yi - fi)^2) / (\Sigma(yi - Y)^2)\}$$

where

i represents a value between j and k, with j and k representing the x coordinates of one and the other ends, respectively, of the contour line or the approximate curve on the xy plane;
yi represents the y coordinate of the point on the contour line at the x coordinate i on the xy plane;
fi represents the y coordinate of the point on the approximate curve at the x coordinate i on the xy plane; and
Y represents the average value of yi for all points on the contour line.

Here, i, j, and k are all integers, fulfilling $j < k$ and $j \le i \le k$. $\Sigma(yi - fi)^2$ represents the sum of $(yi - fi)^2$ as i is varied from j to k, and $\Sigma(yi - Y)^2$ represents the sum of $(yi - Y)^2$ as i is varied from j to k.

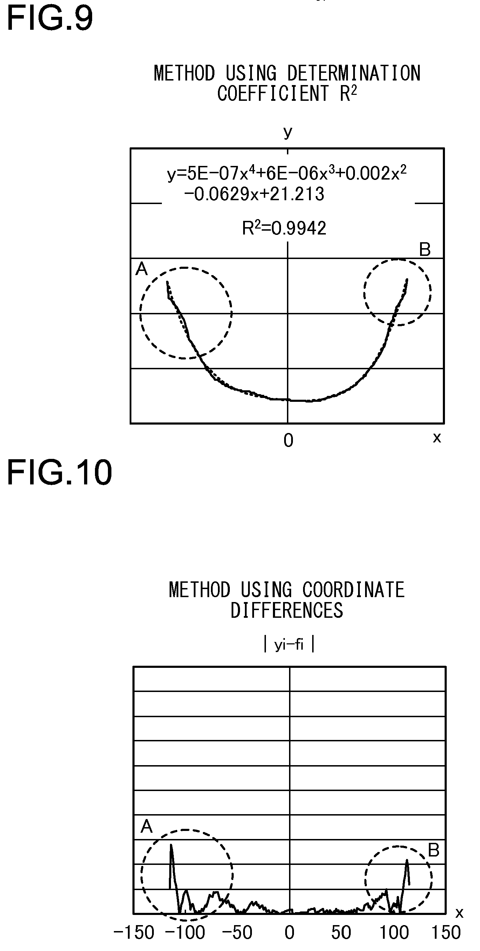

Fig. 9 shows the tongue contour line in Fig. 8, an approximate curve that approximates it, the polynomial equation that expresses the approximate curve, and the determination coefficient $R^2$. The approximate curve is found by the least-square method, and is expressed by the polynomial equation below. Here, the determination coefficient $R^2$ equals 0.9942.

$$y = 5 \times 10^{-7} \cdot x^4 + 6 \times 10^{-6} \cdot x^3 + 2 \times 10^{-3} \cdot x^2 + 6.29 \times 10^{-2} \cdot x + 21.213$$

Both in part A, with a severe tooth mark (large dents and bumps), and in part B, with a mild tooth mark (small dents and bumps), the differences between the contour line and the approximate curve are reflected in the determination coefficient $R^2$. Accordingly, by use of a method that detects a tooth mark based on the determination coefficient $R^2$, even with only a mild tooth mark, it is possible to detect it based on the determination coefficient $R^2$, leading to improved tooth-mark detection precision.

A second index that indicates the degree of correlation is a value obtained based on differences in coordinates (y coordinates) between the contour line and the approximate curve, and is, specifically, the maximum value of |yi - fi| (hereinafter also referred to as the "maximum value of coordinate differences").

Here,

i represents a value between j and k, with j and k representing the x coordinates of one and the other ends, respectively, of the contour line or the approximate curve on the xy plane;

yi represents the y coordinate of the point on the contour line at the x coordinate i

on the xy plane; and

fi represents the y coordinate of the point on the approximate curve at the x coordinate i on the xy plane.

Here, i, j, and k are all integers, fulfilling $j < k$ and $j \leq i \leq k$.

Fig. 10 is a plot of y-coordinate differences (|yi - fi|) between the tongue contour line in Fig. 8 and its approximate curve (an xy polynomial equation). Both in part A, with a severe tooth mark, and in part B, with a mild tooth mark, the value of |yi - fi| is greater than elsewhere, with no tooth mark. Thus, even with only a mild tooth mark, it is possible to detect it, by detecting the maximum value of |yi - fi|, based on this value, leading to improved tooth-mark detection precision.

A third index that indicates the degree of correlation is a value obtained based on differences in coordinates (y coordinates) between the contour line and the approximate curve, and is a coefficient A given by the formula below.

$$A = \Sigma|yi - fi|$$

where

i represents a value between j and k, with j and k representing the x coordinates of one and the other ends, respectively, of the contour line or the approximate curve on the xy plane;

yi represents the y coordinate of the point on the contour line at the x coordinate i on the xy plane; and

fi represents the y coordinate of the point on the approximate curve at the x coordinate i on the xy plane.

Here, i, j, and k are all integers, fulfilling $j < k$ and $j \leq i \leq k$. $\Sigma|yi - fi|$ represents the sum of |yi — fi| as i is varied from j to k (hereinafter also referred to as the "sum of coordinate differences").

Using the sum of coordinate differences instead of the maximum value of coordinate differences allows tooth-mark detection that accurately reflects the influence of a fine tooth mark, leading to further improved tooth-mark detection precision.

As the degree of correlation, it is also possible to use the reciprocal of the sum of the coordinate differences, namely $1 / (\Sigma|yi - fi|)$.

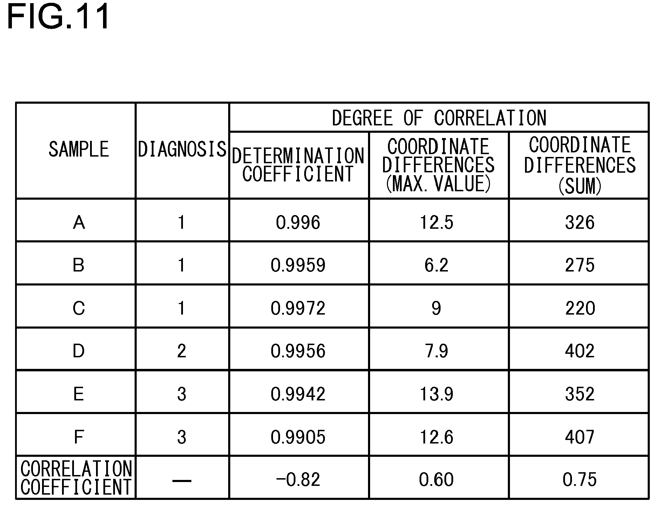

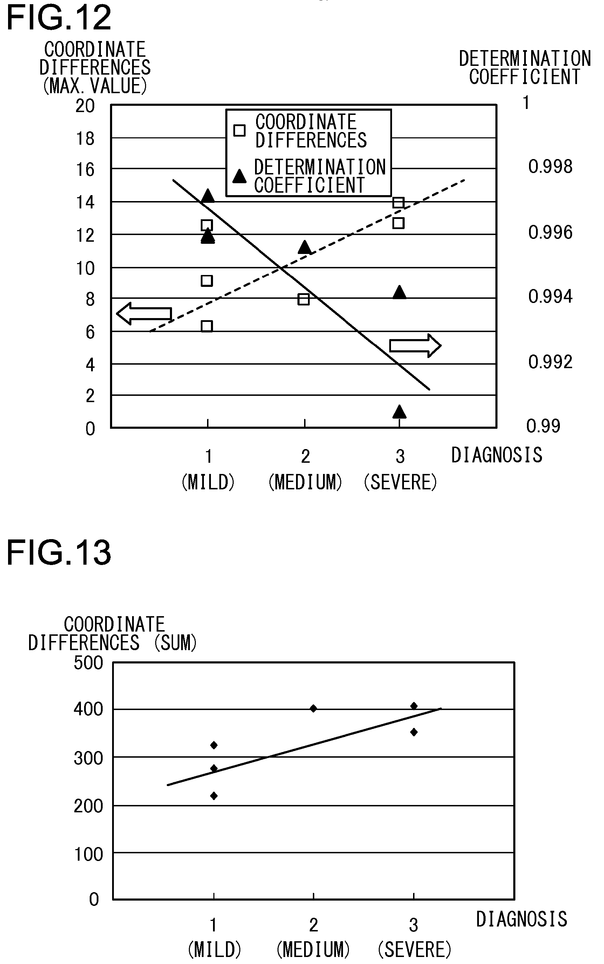

Fig. 11 shows, for the tongues of six individuals, referred to as samples A to F respectively, the relationship between actual diagnoses by a *Kampo* doctor and the degree of correlation discussed above (the determination coefficient $R^2$, the maximum value of coordinate differences, and the sum of coordinate differences). Fig. 12 is a plot of the relationship between the diagnoses by a *Kampo* doctor and the determination coefficient $R^2$ or the maximum value of coordinate differences in Fig. 11, and Fig. 13 is a plot of the relationship between the diagnoses by a *Kampo* doctor and the sum of coordinate differences in Fig. 11. The diagnoses by a *Kampo* doctor are made in three levels: level 1 indicating a mild tooth mark; level 2 indicating a medium tooth mark between a mild and a severe one; level 3 indicating a severe tooth mark.

From Fig. 12, it is understood that there is a correlation between the determination coefficient $R^2$ or the maximum value of coordinate differences, which indicates the degree of correlation, and the *Kampo* doctor's diagnoses. More specifically, there is a negative correlation between the determination coefficient $R^2$ and the *Kampo* doctor's diagnoses: the greater the determination coefficient $R^2$ is, the closer the diagnoses are to level 1 (a mild tooth mark); the smaller the determination coefficient $R^2$ is, the closer the diagnoses are to level 3 (a severe tooth mark). On the other hand, there is a positive correlation between the maximum value of coordinate differences and the *Kampo* doctor's diagnoses: the greater the maximum value of coordinate differences is, the closer the diagnoses are to level 3 (a severe tooth mark); the smaller the maximum value of coordinate differences is, the closer the diagnoses are to level 1 (a mild tooth mark). From Fig. 13, it is understood that there is a positive correlation also between the sum of coordinate differences and the *Kampo* doctor's diagnoses: the greater the sum of coordinate differences is, the closer the diagnoses are to level 3; the smaller the sum of coordinate differences is, the closer the diagnoses are to level 1.

Fig. 12 reveals that the correlation coefficient R that indicates the degree of correlation between the determination coefficient $R^2$ and the diagnoses was -0.82 (the minus sign indicating a negative correlation). Likewise, the correlation coefficient R that indicates the degree of correlation between the maximum value of coordinate differences and the diagnoses was 0.60, and the correlation coefficient R that indicates the degree of correlation between the sum of coordinate differences and the diagnoses was 0.75. From these results, it can be concluded that, when used as the degree of correlation, the maximum value of coordinate differences yields results close to diagnoses by a *Kampo* doctor, the sum of coordinate differences yields results closer to diagnoses by a *Kampo* doctor, and the determination coefficient $R^2$ yields results closest to diagnoses by a *Kampo* doctor. The correlation coefficient R equals the square root of the result of calculation according to the earlier-noted equation of the determination coefficient $R^2$ with the determination coefficient, the maximum value of coordinate differences, or the sum of coordinate differences taken as a sample value yi, and a point on the regression line taken as an estimated value fi.

With the relationship between a *Kampo* doctor's diagnoses and different degrees of correlation stored in the storage 8 in the form of a table as shown in Fig. 11 or in the form of a plot as shown in Figs. 12 and 13, the detector 7 can detect a tooth mark based on the degree of correlation, and can also determine the severity of a tooth mark (e.g. whether it is severe or mild) based on the information (diagnoses corresponding to different degrees of correlation).

[Control Flow]

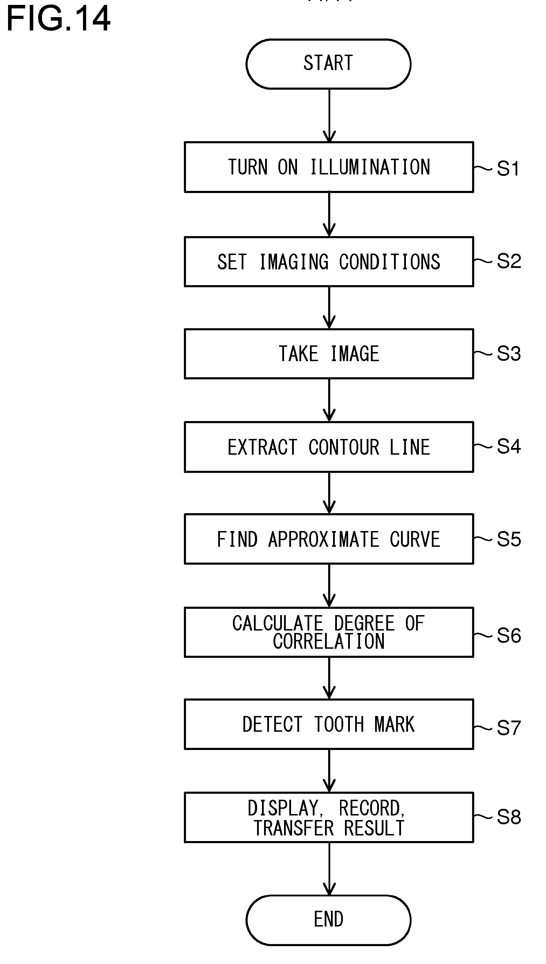

Fig. 14 is a flow chart showing the flow of operation in the organ imaging device 1 according to Embodiment 1. In response to an instruction to take an image from the operation panel 5 or from an unillustrated input device, the controller 10 lights the illuminator 2 (S1), and sets imaging conditions (S2). On completion of the setting of imaging conditions, the controller 10 controls the imager 3 so as to take an image of the tongue as an imaging object (S3).

On completion of the imaging, the contour line extractor 6 extracts a contour line of the tongue from the taken image of the tongue (S4). Then, from the extracted contour line, the detector 7 finds an approximate curve approximating it by the least-square method, and calculates the degree of correlation between the contour line and the approximate curve (a value based on a determination coefficient $R^2$ or based on coordinate differences) (S6). The finding of the approximate curve and the calculation of the degree of correlation may be performed concurrently. Specifically, at Step S5, an approximate curve that gives the highest degree of correlation may be searched for while the degree of correlation is being calculated.

Then, based on the degree of correlation found at Step S6, the detector 7 checks for a tooth mark (dents and bumps in the contour line) and its severity (degree) while referring to the table stored in the storage 8 (S7). Thus, based on the result of the detection, the detector 7 allows diagnosis of the healthiness of the examinee. The detection result may be quantified and transmitted to outside so that the examinee's healthiness may be diagnosed outside. The result of tooth-mark detection and the result of the diagnosis of the examinee's healthiness are displayed on the display 4, and are, as necessary, output (recorded) to an unillustrated output device or transferred to outside via the communicator 9 (S8).

As described above, the detector 7 finds an approximate curve that approximates the contour line of the tongue extracted by the contour line extractor 6, and detects dents and bumps in the contour line based on the degree of correlation between the contour line and the approximate curve. The approximate curve, being approximate to the tongue contour line, reflects the exterior shape of the tongue (see Fig. 6). Thus, by detecting dents and bumps in the contour line based on the degree of correlation between the contour line and the approximate curve, whatever exterior shape the tongue may have, it is possible to detect dents and bumps in the contour line with reference to the approximate curve for each individual shape, and to precisely detect dents and bumps in the contour line irrespective of the exterior shape of the tongue. Moreover, even in a case where the tongue contour line has only small dents and bumps, the degree of correlation is determined with consideration given to such dents and bump, and thus, based on the determined degree of correlation, it is possible to precisely detect those dents and bumps.

As described above, irrespective of the exterior shape of an organ, and even in a case where the contour line only has small dents and bumps, it is possible to precisely detect the dents and bumps in the contour line. This helps enhance the precision of diagnosis based on dents and bumps in a contour line.

Moreover, the above-mentioned approximate curve is expressed by the polynomial equation with the highest degree of correlation with the contour line out of polynomial equations of the same maximum degree. Thus, with reference to the approximate curve closest to the contour line out of polynomial equations of the same maximum degree, it is possible to more precisely detect dents and bumps in the contour line.

In a case where the organ as an imaging object is the tongue, by detecting dents and bumps in the contour line, the detector detects a tooth mark. Thus, irrespective of the exterior shape of the tongue, and even in a case where the tooth mark is mild, it is possible to precisely detect the tooth mark.

Moreover, with the approximate curve expressed by a polynomial equation of degree 4 or more, whatever exterior shape an organ may have, it is possible to reliably approximate the contour line of the organ with the approximate curve. This helps reliably enhance the precision of the detection of dents and bumps in the contour line based on the degree of correlation between the organ's contour line and the approximate curve.

Moreover, by using as the degree of correlation a determination coefficient $R^2$ or a value based on coordinate differences (the maximum value or the sum of |yi - fi|) as mentioned above, it is possible to properly express the correlation between the contour line and the approximate curve. It is thus possible to reliably obtain the effect of Embodiment 1, namely the effect of enhancing the precision of the detection of dents and bumps in a contour line based on a degree of correlation.

<Embodiment 2>

Next, another embodiment of the present invention will be described with reference to the accompanying drawings. The following description focuses on differences from Embodiment 1.

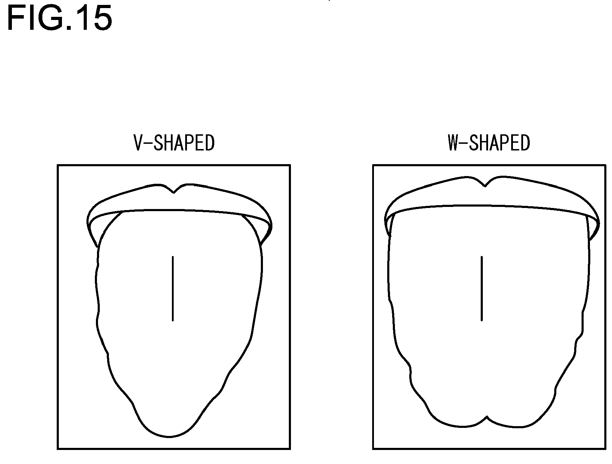

Fig. 15 shows other examples of taken images of the tongue. As shown there, a tip part of the tongue can have varying shapes, including a V shape with a pointed tip and a W shape with divided tips on the left and right sides respectively. While the tip of the tongue has varying shapes from one individual to another, in general, when the tongue is tense, it tends to have a V-shaped tip part. On the other hand, the intrinsic muscle of the tongue is divided into a left and a right part along the lingual septum extending longitudinally along the median line of the tongue. With the muscle well-developed in those parts, the tongue tends to have a W-shaped tip part.

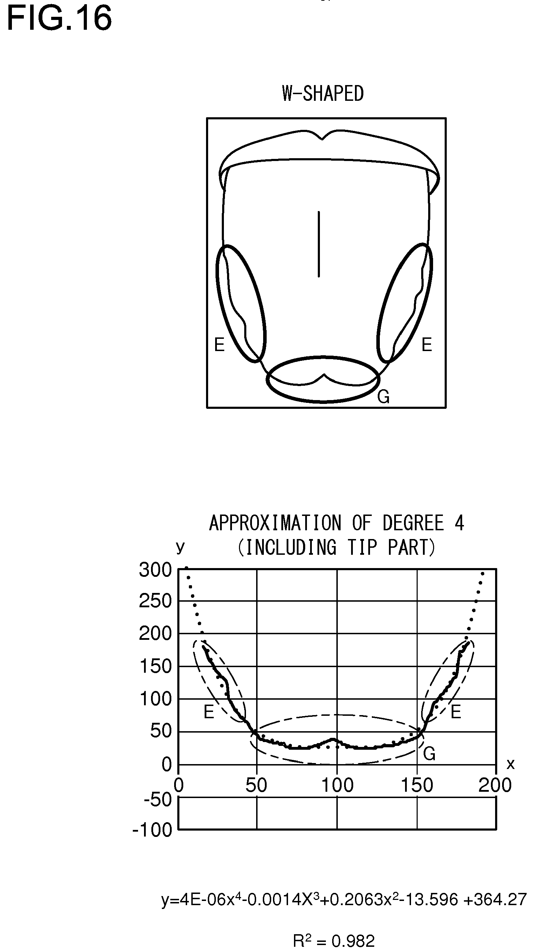

Fig. 16 shows, for example for a case where the tongue has a W-shaped tip part, the tongue contour line and its approximate curve. The approximate curve here represents the lower half of the extracted tongue contour line as approximated by a polynomial equation of order 4. In Fig. 16, the tongue contour line is indicated by a solid line, and the approximate curve approximating it is indicated by a broken line. The approximate curve here is given by the following equation.

$$y = 4 \times 10^{-6} \cdot x^4 - 1.4 \times 10^{-3} \cdot x^3 + 2.063 \times 10^{-1} \cdot x^2 - 13.596 \cdot x + 364.27$$

Divergences between the tongue contour line (solid line) and the approximate curve (broken line) are observed not only in tooth-mark parts (parts E in Fig. 16), i.e., parts to be detected, located in a left and a right part of the tongue but also in a W-shaped tip part (part G in Fig. 16). Accordingly, if the contour line of the tongue including its tip part is approximated by an approximate curve, the degree of correlation between the contour line and the approximate curve may be lower than it should be, leading to an incorrect diagnosis of more tooth marks than there actually are. Incidentally, in Fig. 16, the determination coefficient $R^2$ indicating the degree of correlation between the tongue contour line and the approximate curve was 0.982.

As a solution, in Embodiment 2, the detector 7 approximates, out of the tongue contour line extracted by the contour line extractor 6, a part excluding a tip part with an approximate curve, and detects a tooth mark (dents and bumps in the contour line) based on the degree of correlation between the two. Here, a tip part of the tongue denotes a part of it corresponding to, assuming that the tip end of the tongue is at position 0 and the base end of the tongue is at position 100, a range from 0 to 20, and more preferably a range from 0 to 10.

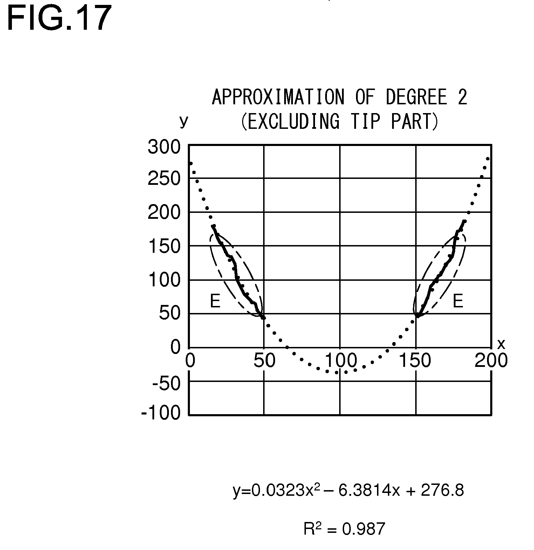

Fig. 17 shows a part (excluding a tip part) of the tongue contour line extracted from the taken image of the tongue shown in Fig. 16 and an approximate curve approximating it. In Fig. 17, of the lower half of the extracted contour line, a part excluding a tip part (50 to 150 along the x axis) is approximated by a polynomial equation of degree 2. In Fig. 17, part of the tongue contour line is indicated by a solid line, and its approximate curve is indicated by a broken line. The found approximate curve (approximating equation) is given by the following equation.

$$y = 3.23 \times 10^{-2} \cdot x^2 - 6.3814 \cdot x + 276.8$$

Divergences between the part (solid line) of the tongue contour line and the approximate curve (broken line) are limited to the tooth-mark parts (parts E in Fig. 17), i.e., the parts to be detected, located in a left and a right part of the tongue, and are not influenced by the shape of the tip part of the tongue (part G in Fig. 16). Accordingly, the degree of correlation between the two indicates just the size of the tooth marks in a left and a right part of the tongue. Incidentally, in Fig. 17, finding a determination coefficient $R^2$ that indicates the degree of correlation between the part of the tongue contour line and the approximate curve yields $R^2 = 0.987$, which is a higher degree of correlation than when the approximate curve shown in Fig. 16 was used. The difference between the degrees of correlation in Figs. 16 and 17 results from an error ascribable to the shape at the tongue tip, and the degree of correlation found by use of the approximate curve shown in Fig. 17 reflects the condition of tooth mark more accurately.

In Embodiment 2, as an index that indicates the degree of correlation, a determination coefficient $R^2$ described in connection with Embodiment 1 is used; instead, a value based on coordinate differences (the maximum value of $|y_i - f_i|$, a coefficient A), likewise described in connection with Embodiment 1, may be used. In that case, the determination coefficient $R^2$ and the value based on coordinate differences can be calculated according to the equations noted in connection with Embodiment 1 assuming that "the x coordinate of one end of the contour line or approximate curve on the xy plane" denotes "the minimum x coordinate of the contour line or approximate curve on the xy plane", and that "the x coordinate of the other end of the contour line or approximate curve on the xy plane" denotes "the maximum x coordinate of the contour line or approximate curve on the xy plane".

As described above, in Embodiment 2, the detector 7 finds an approximate curve that approximates a part of the contour line of the tongue excluding a tip part thereof, and detects dents and bumps in the contour line based on the degree of correlation between the part of the contour line and the approximate curve. As mentioned above, a tip part of the tongue has varying shapes, like a V shape and a W shape, due to causes other than those intended to be diagnosed such as poor water metabolism. By detecting dents and bumps in the contour line based on the degree of correlation between a part of the tongue contour line excluding a tip part and the approximate curve as in Embodiment 2, it is possible, without being influenced by the shape of the tip part of the tongue (while eliminating the influence of tenseness and differences among individuals), to detect dents and bumps (tooth marks) resulting from poor water metabolism, which is a cause intended to be diagnosed. It is thus possible to reduce the inconvenience of the degree of correlation falling down under the influence of the shape of the tip part of the tongue and leading to an incorrect diagnosis of more dents and bumps than there actually are, and thus to make a more precise diagnosis based on dents and bumps in the contour line.

Moreover, the above-mentioned approximate curve is expressed by a polynomial equation of degree 2. Thus, compared with Embodiment 1, where the tongue contour line is approximated by a polynomial equation of degree 4, it is possible to reduce the degree of the approximate curve. This helps reduce the calculation load for the finding of the approximate curve by the detector 7, and thus helps achieve cost reduction and higher processing speed.

[Other]

Although the above description deals with a case where the imaging object is the human tongue, the living body (anything alive) does not necessarily have to be a human but may be any animal other than a human. For example, also with the tongue of a pet or other domestic animal, the procedure according to the embodiment can be used to detect dents and bumps in the contour line of the tongue, and to make a diagnosis based on the detected dents and bumps. In that way, it is possible to recognize poor health condition of an animal, which cannot communicate by words.

Moreover, the organ of a living body that can be taken as an imaging object is not limited to the tongue. For example, it can instead be the lips, or a part inside the oral cavity, such as the gum, or the lining of the stomach or the intestines. By detecting dents and bumps in a contour line of one of those organs (in the case of the lining of the stomach or the intestines, a contour line of folds in the lining), it is possible to detect erosion that has developed in an organ, and to make a diagnosis based on the result of the detection. It is also possible to detect dents and bumps in a contour line simultaneously when checks are made in respective medical specialties.

Although in the embodiments described above, a determination coefficient $R^2$ or a value based on coordinate differences is used as the degree of correlation between a contour line and an approximate curve, it is possible to use instead a correlation coefficient R, which is the square root of the determination coefficient $R^2$, and to detect dents and bumps in the contour line based on the correlation coefficient R.

An organ imaging device described herein can be said to be configured as noted below, and provides benefits as noted below.

As described herein, an organ imaging device including an imager for imaging an organ of a living body to acquire an image of the organ is configured to include: a contour line extractor for extracting a contour line of the organ from the image acquired by the imager; and a detector for finding an approximate curve that approximates the contour line, or part of the contour line, of the organ extracted by the contour line extractor, and detecting dents and bumps in the contour line based on the degree of correlation between the contour line and the approximate curve. The approximate curve may be one that approximates the contour line of the organ including a tip part of the organ, or may be one that approximates part of the contour line (e.g., a part excluding a tip part of the organ).

The contour line of the organ extracted by the contour line extractor has a shape that largely fits the exterior shape of the organ. Thus, by letting the detector find an approximate curve that approximates the contour line, or part of it, it is possible to obtain an approximate curve that reflects the exterior shape of the organ. Then, by detecting dents and bumps in the contour line based on the degree of correlation between the contour line of the organ and the approximate curve, it is possible to detect the degree of dents and bumps in the contour line with reference to the approximate curve, that is, the exterior shape of the organ. In this way, whatever exterior shape the organ may have, it is possible to detect the degree of dents and bumps in the contour line in a way that suits the specific exterior shape. It is thus passible to precisely detect dents and bumps in the contour line irrespective of the exterior shape of the organ.

Moreover, even in a case where the contour line has only such dents and bumps as are so fine as not to produce shadings, the degree of correlation between the contour line and the approximate curve is determined with those dents and bumps taken into consideration. Thus, by letting the detector detect dents and bumps in the contour line based on that degree of correlation, it is possible, even in a case where the contour line has only small dents and bumps, to precisely detect those dents and bumps based on the degree of correlation.

The approximate curve may be expressed by, out of polynomial equations of the same maximum degree, the polynomial equation that yields the highest degree of correlation with the contour line. Thus, it is possible to more precisely detect dents and bumps in the contour line with reference to the polynomial equation (approximate curve) that is closest to the contour line among polynomial equations with the same maximum degree.

The organ may be a tongue, and the detector may detect the dents and bumps in the contour line to detect a tooth mark on the tongue. Thus, it is possible to precisely detect the tooth mark irrespective of the exterior shape of the tongue and even in a case where the tooth mark is mild.

It is preferable that the detector detect the tooth mark on the tongue by referring to a relationship between a previously stored diagnosis by a *Kampo* doctor and the degree of correlation. Thus, the detector can determine the severity of the detected tooth mark (e.g., whether it is severe or mild) based on a diagnosis by a *Kampo* doctor corresponding to the degree of correlation.

The detector may find an approximate curve that approximates part of the contour line excluding a tip part of the tongue, and may detect dents and bumps in the contour line based on the degree of correlation between the part of the contour line and the approximate curve. Thus, it is possible to more precisely detect dents and bumps in the contour line without being influenced by the shape of a tip part of the tongue, which tends to vary with tenseness or among individuals. This helps enhance the precision of a diagnosis based on dents and bumps in the contour line.

The approximate curve may be expressed by a polynomial equation of degree 4 or more. Thus, it is possible to reliably approximate the contour line of the organ with an approximate curve whatever exterior shape the organ may have. It is thus possible to precisely and reliably detect dents and bumps in the contour line.

The approximate curve may be expressed by a polynomial equation of degree 2. The approximate curve then has a maximum degree of 2. This helps reduce the calculation load for finding the approximate curve (compared with when finding an equation of degree 4), and thus helps achieve cost reduction and high processing speed.

The degree of correlation may be a determination coefficient $R^2$ given by the following equation:

$$R^2 = 1 - \{(\Sigma(yi - fi)^2) / (\Sigma(yi - Y)^2)\}$$

where

i represents a value between j and k, with j and k representing the x coordinates of one and the other ends, respectively, of the contour line or the approximate curve on the xy plane;
yi represents the y coordinate of the point on the contour line at the x coordinate i on the xy plane;
fi represents the y coordinate of the point on the approximate curve at the x coordinate i on the xy plane; and
Y represents the average value of yi for all points on the contour line.

By using the determination coefficient $R^2$ noted above as the degree of correlation, it is possible to properly express the correlation between the contour line and the approximate curve. It is thus possible to reliably obtain the effect of enhancing the precision of the detection of dents and bumps in the contour line based on the degree of correlation.

The degree of correlation may be a value based on coordinate differences between the contour line and the approximate curve. For example, the value based on coordinate differences may be the maximum value of |yi - fi| where

i represents a value between j and k, with j and k representing the x coordinates of one and the other ends, respectively, of the contour line or the approximate curve on the xy plane;
yi represents the y coordinate of the point on the contour line at the x coordinate i on the xy plane; and
fi represents the y coordinate of the point on the approximate curve at the x coordinate i on the xy plane.

For another example, the value based on coordinate differences may be a coefficient A given by the following equation:

$$A = \Sigma|yi - fi|$$

where

i represents a value between j and k, with j and k representing the x coordinates of one and the other ends, respectively, of the contour line or the approximate curve on the xy plane;

yi represents the y coordinate of the point on the contour line at the x coordinate i on the xy plane; and

fi represents the y coordinate of the point on the approximate curve at the x coordinate i on the xy plane.

By using the maximum value of |yi - fi| or the coefficient A as the degree of correlation, it is possible to properly express the correlation between the contour line and the approximate curve. It is thus possible to reliably obtain the effect of enhancing the precision of the detection of dents and bumps in the contour line based on the degree of correlation.

**Industrial Applicability**

The present invention finds applications in devices that detect dents and bumps in a contour line of an organ of a living body from an image obtained by imaging the organ.

**List of Reference Signs**

1     organ imaging device
3     imager
6     contour line extractor
7     detector

**Claims**

1.  An organ imaging device including an imager for imaging an organ of a living body to acquire an image of the organ, the organ imaging device comprising:

    a contour line extractor for extracting a contour line of the organ from the image acquired by the imager; and
    a detector for

      finding an approximate curve that approximates the contour line, or part of the contour line, of the organ extracted by the contour line extractor and
      detecting dents and bumps in the contour line based on a degree of correlation between the contour line and the approximate curve.

2.  The organ imaging device of claim 1, wherein
    the approximate curve is expressed by, out of polynomial equations of a same maximum degree, a polynomial equation that yields a highest degree of correlation with the contour line.

3.  The organ imaging device of claim 1 or 2, wherein
    the organ is a tongue, and
    the detector detects the dents and bumps in the contour line to detect a tooth mark on the tongue.

4.  The organ imaging device of claim 3, wherein
    the detector detects the tooth mark on the tongue by referring to a relationship between a previously stored diagnosis by a *Kampo* doctor and the degree of correlation.

5.  The organ imaging device of claim 3 or 4, wherein
    the detector finds an approximate curve that approximates part of the contour line excluding a tip part of the tongue, and detects the dents and bumps in the contour line based on the degree of correlation between the part of the contour line and the approximate curve.

6.  The organ imaging device of any one of claims 1 to 4, wherein
    the approximate curve is expressed by a polynomial equation of degree 4 or more.

7.  The organ imaging device of claim 5, wherein

the approximate curve is expressed by a polynomial equation of degree 2.

8. The organ imaging device of any one of claims 1 to 7, wherein
   the degree of correlation is a determination coefficient $R^2$ given by the following equation:

$$R^2 = 1 - \{(\Sigma(yi - fi)^2) / (\Sigma(yi - Y)^2)\}$$

where

   i represents a value between j and k, with j and k representing x coordinates of one and another ends, respectively, of the contour line or the approximate curve on the xy plane;
   yi represents a y coordinate of a point on the contour line at an x coordinate i on the xy plane;
   fi represents a y coordinate of a point on the approximate curve at an x coordinate i on the xy plane; and
   Y represents an average value of yi for all points on the contour line.

9. The organ imaging device of any one of claims 1 to 7, wherein
   the degree of correlation is a value based on coordinate differences between the contour line and the approximate curve.

10. The organ imaging device of claim 9, wherein
    the value based on coordinate differences is a maximum value of |yi - fi|
    where
    i represents a value between j and k, with j and k representing x coordinates of one and another ends, respectively, of the contour line or the approximate curve on the xy plane;
    yi represents a y coordinate of a point on the contour line at an x coordinate i on the xy plane; and
    fi represents a y coordinate of a point on the approximate curve at an x coordinate i on the xy plane

11. The organ imaging device of clainm9, wherein
    the value based on coordinate differences is a coefficient A given by the following equation:

$$A = \Sigma|yi - fi|$$

where

   i represents a value between j and k, with j and k representing x coordinates of one and another ends, respectively, of the contour line or the approximate curve on the xy plane;
   yi represents a y coordinate of a point on the contour line at an x coordinate i on the xy plane; and
   fi represents a y coordinate of a point on the approximate curve at an x coordinate i on the xy plane.

FIG.1

# FIG.2

1

| | | |
|---|---|---|
| ILLUMINATOR | | CONTOUR LINE EXTRACTOR |
| 2 | | 6 |
| IMAGER | | DETECTOR |
| 3 | CONTROLLER | 7 |
| DISPLAY | | STORAGE |
| 4 | 10 | 8 |
| OPERATION PANEL | | COMMUNICATOR |
| 5 | | 9 |

# FIG.3

TAKEN IMAGE

4

EDGE EXTRACTION FILTER

| | −1 | |
|---|---|---|
| −1 | 4 | −1 |
| | −1 | |

EXTRACTED CONTOUR
LINE

FIG.4

# FIG.5

APPROXIMATE CURVE OF DEGREE 2    APPROXIMATE CURVE OF DEGREE 3    APPROXIMATE CURVE OF DEGREE 4

$$y=0.0074x^2-0.0161x+14.005$$

$$R^2=0.9475$$

$$y=6E-06x^3+0.0074x^2-0.0629x+14.005$$

$$R^2 = 0.9495$$

$$y=5E-07x^4+6E-06x^3+0.002x^2-0.0629x+21.213$$

$$R^2=0.9942$$

EP 3 061 395 A1

FIG.6

# FIG.7

# FIG.8

# FIG.9

METHOD USING DETERMINATION
COEFFICIENT $R^2$

y

$y=5E{-}07x^4+6E{-}06x^3+0.002x^2$
$-0.0629x+21.213$

$R^2=0.9942$

B

A

0      x

# FIG.10

METHOD USING COORDINATE
DIFFERENCES

$|\ y_i{-}f_i\ |$

A

B

x

$-150$   $-100$   $-50$    0     50    100   150

FIG.11

| SAMPLE | DIAGNOSIS | DEGREE OF CORRELATION | | |
|---|---|---|---|---|
| | | DETERMINATION COEFFICIENT | COORDINATE DIFFERENCES (MAX. VALUE) | COORDINATE DIFFERENCES (SUM) |
| A | 1 | 0.996 | 12.5 | 326 |
| B | 1 | 0.9959 | 6.2 | 275 |
| C | 1 | 0.9972 | 9 | 220 |
| D | 2 | 0.9956 | 7.9 | 402 |
| E | 3 | 0.9942 | 13.9 | 352 |
| F | 3 | 0.9905 | 12.6 | 407 |
| CORRELATION COEFFICIENT | — | −0.82 | 0.60 | 0.75 |

FIG.12

FIG.13

# FIG.14

```
          ┌─────────────┐
          │    START    │
          └──────┬──────┘
                 │
                 ▼
    ┌────────────────────────┐
    │  TURN ON ILLUMINATION   │──── S1
    └────────────┬───────────┘
                 │
                 ▼
    ┌────────────────────────┐
    │  SET IMAGING CONDITIONS │──── S2
    └────────────┬───────────┘
                 │
                 ▼
    ┌────────────────────────┐
    │       TAKE IMAGE        │──── S3
    └────────────┬───────────┘
                 │
                 ▼
    ┌────────────────────────┐
    │   EXTRACT CONTOUR LINE  │──── S4
    └────────────┬───────────┘
                 │
                 ▼
    ┌────────────────────────┐
    │  FIND APPROXIMATE CURVE │──── S5
    └────────────┬───────────┘
                 │
                 ▼
    ┌────────────────────────┐
    │   CALCULATE DEGREE OF   │──── S6
    │      CORRELATION        │
    └────────────┬───────────┘
                 │
                 ▼
    ┌────────────────────────┐
    │    DETECT TOOTH MARK    │──── S7
    └────────────┬───────────┘
                 │
                 ▼
    ┌────────────────────────┐
    │   DISPLAY,  RECORD,     │──── S8
    │  TRANSFER RESULT        │
    └────────────┬───────────┘
                 │
                 ▼
          ┌─────────────┐
          │     END     │
          └─────────────┘
```

# FIG.15

V-SHAPED        W-SHAPED

# FIG.16

W-SHAPED

APPROXIMATION OF DEGREE 4
(INCLUDING TIP PART)

$y=4E-06x^4-0.0014X^3+0.2063x^2-13.596+364.27$

$R^2 = 0.982$

# FIG.17

APPROXIMATION OF DEGREE 2
(EXCLUDING TIP PART)

$y = 0.0323x^2 - 6.3814x + 276.8$

$R^2 = 0.987$

## INTERNATIONAL SEARCH REPORT

| International application No. |
| --- |
| PCT/JP2014/075876 |

| A. CLASSIFICATION OF SUBJECT MATTER |
| --- |
| *A61B5/107(2006.01)i, G06T1/00(2006.01)i* |

According to International Patent Classification (IPC) or to both national classification and IPC

| B. FIELDS SEARCHED |
| --- |
| Minimum documentation searched (classification system followed by classification symbols)<br>A61B5/107, G06T1/00 |

Documentation searched other than minimum documentation to the extent that such documents are included in the fields searched

| Jitsuyo Shinan Koho | 1922–1996 | Jitsuyo Shinan Toroku Koho | 1996–2014 |
| --- | --- | --- | --- |
| Kokai Jitsuyo Shinan Koho | 1971–2014 | Toroku Jitsuyo Shinan Koho | 1994–2014 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| A | JP 2009-028058 A  (Kabushiki Kaisha saieco),<br>12 February 2009 (12.02.2009),<br>entire text; all drawings<br>(Family: none) | 1-11 |
| A | JP 2005-137756 A  (Konica Minolta Holdings, Inc.),<br>02 June 2005 (02.06.2005),<br>entire text; all drawings<br>(Family: none) | 1-11 |
| A | KR 10-0865275 B1  (Korea Institute of Oriental Medicine),<br>24 October 2008 (24.10.2008),<br>entire text; all drawings<br>(Family: none) | 1-11 |

☒ Further documents are listed in the continuation of Box C.　　☐ See patent family annex.

| * | Special categories of cited documents: |
| --- | --- |
| "A" | document defining the general state of the art which is not considered    to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>　　18 November, 2014 (18.11.14) | Date of mailing of the international search report<br>　　02 December, 2014 (02.12.14) |
| --- | --- |
| Name and mailing address of the ISA/<br>　　Japanese Patent Office | Authorized officer |
| Facsimile No. | Telephone No. |

Form PCT/ISA/210 (second sheet) (July 2009)

**INTERNATIONAL SEARCH REPORT**

| International application No. |
| --- |
| PCT/JP2014/075876 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 2005-296349 A (Nissan Motor Co., Ltd.), 27 October 2005 (27.10.2005), entire text; all drawings (Family: none) | 1-11 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005137756 A

- JP 2009028058 A